# EUROPÄISCHE PATENTSCHRIFT

(11) **EP 1 612 160 B1**
(45) Veröffentlichungstag und Bekanntmachung des Hinweises auf die Patenterteilung: **23.08.2006**
(21) Anmeldenummer: 05008259.3
(22) Anmeldetag: 15.04.2005
(51) Int. Cl.: B65D 83/08, A61F 15/00

(54) **Behälter zum Aufnehmen und Entsorgen eines Hygieneartikels**
Dispensing and disposal container for hygiene items
Contenant-distributeur et pot à déchet pour produits hygiéniques

(30) Priorität: 01.06.2004 DE 102004026954; 20.08.2004 DE 102004040463
(43) Veröffentlichungstag der Anmeldung: 04.01.2006
(73) Patentinhaber: Weinmann, Gerold, Dr., 59514 Welver (DE)
(72) Erfinder: Weinmann, Gerold, Dr., 59514 Welver (DE)
(74) Vertreter: Bittner, Thomas L.

(56) Entgegenhaltungen:
- WO-A-02/17844
- DE-A1- 10 061 670
- DE-U1- 20 103 539
- US-A- 3 245 580
- US-A- 4 564 108
- US-A- 4 879 442
- US-A1- 2003 178 436

## Beschreibung

Die Erfindung betrifft eine Vorrichtung zum Aufnehmen eines zu entsorgenden Hygieneartikels, insbesondere Damen-Tampon.

Derartige Vorrichtungen werden benutzt, um insbesondere Einweg-Hygieneartikel nach deren Benutzung für eine Entsorgung aufzunehmen. Bei solchen Einweg-Hygieneartikeln handelt es sich beispielsweise um Damen-Tampons. Hierbei sind die Hygieneartikel nicht nur hygienisch und geruchsneutral bis zur Entsorgung zu lagern, es besteht auch Bedarf für eine einfache Handhabung bei der Benutzung der Vorrichtung.

Aus dem Dokument US 2,915,218 ist ein Behälter bekannt, welcher einen Teilbehälter mit unverbrauchten Papiertaschentüchern sowie eine weiteren Teilbehälter zum Aufnehmen verbrauchter Papiertaschentücher umfaßt. Der weitere Teilbehälter zum Aufnehmen der verbrauchten Papiertaschentücher verfügt hierbei über eine verschließbare Öffnung, durch die die verbrauchten Papiertaschentücher eingeworfen werden können.

Ähnliche Behälter für Papiertaschentücher, die über einen Papiertaschentuchspender und einen Teilbehälter zur Aufnahme verbrauchter Papiertaschentücher verfügen, sind in den Dokumenten DE 100 61 670 A1 und US 4,879,442 beschrieben.

Darüber hinaus sind Spender bekannt, mit denen ein Vorrat an Hygieneartikeln, beispielsweise Windeln, Damenbinden oder dergleichen, zur stückweisen Entnahme zur Verfügung gestellt ist. Solche Spender sind in den Dokumenten US 3,245,580 sowie DE 201 03 539 U1 beschrieben.

Es ist weiterhin bekannt, Damenbinden vor dem Gebrauch bei deren Verpacken für den anschließenden Vertrieb und Verkauf einzeln mit einer Umverpackung zu versehen, wie dieses in dem Dokument DE 34 21 957 A1 erläutert ist.

Aus dem Dokument US 2003/0178436 ist eine Vorrichtung mit Öffnungen zum Entnehmen von unbenutzten Tüchern sowie einer weiteren Öffnung für zu entsorgende, gebrauchte Tücher bekannt. Ferner umfaßt die Vorrichtung Behälter mit unbenutzten Tüchern, welche durch die Öffnungen entnommen werden können und einen weiteren Behälter, in welchem die gebrauchten Tücher durch die weitere Öffnung zur Entsorgung eingeworfen werden können.

Das Dokument WO 0 217 844 offenbart einen Behälter aus mehreren Teilbehältern, welche als Vorratsräume für unterschiedliche unbenutzte Hygieneartikel dienen.

Aufgabe der Erfindung ist es deshalb, eine Vorrichtung zum Aufnehmen eines zu entsorgenden Hygieneartikels anzugeben, der einen hygienischen Umgang mit dem zu entsorgenden Hygieneartikel und eine für den Benutzer bequeme Handhabung ermöglicht. Darüber hinaus soll die Vorrichtung kostengünstig herstellbar sein.

Diese Aufgabe wird erfindungsgemäß durch eine Vorrichtung gemäß Anspruch 1 gelöst.

Mit Hilfe der Vorrichtung wird einerseits eine Möglichkeit zum Zwischenlagern des gebrauchten Hygieneartikels in dem Aufnahmeraum bis zur tatsächlichen Entsorgung geschaffen. Andererseits ist in dem Behälter ein von dem Aufnahmeraum getrennten Vorratsraum vorgesehen, in dem Verpackungen gelagert werden, die zur Aufnahme des zu entsorgenden Hygieneartikels vor dem Einwerfen in den Aufnahmeraum dienen. Auf diese Weise wird der Hygienestandard verbessert, da der zu entsorgende Hygieneartikel in dem Aufnahmeraum von der Verpackung umgeben ist. Hierdurch wird weiterhin eine Geruchsbelästigung am Aufstellort der Vorrichtung vermieden. Es ist eine integrierte Lösung sowohl zum Aufnehmen des zu entsorgenden Hygieneartikels als auch zur Bereitstellung von Verpackungen für den Hygieneartikel zur Verfugung gestellt, die an beliebigen Orten verwendet werden kann.

Eine zweckmäßige Ausgestaltung der Erfindung sieht einen Deckel vor, der zum Verschlie-ßen und zum Freigeben der Einwurföffnung zwischen einer geschlossenen und einer geöffneten Stellung schwenkbar ist. Der Deckel kann aus einem Kunststoffmaterial sein. Mit Hilfe des Deckels wird die Einwurföffnung bei Nichtbenutzung verschlossen, so daß der Aufnahmeraum mit zu entsorgenden Hygieneartikeln abgeschlossen ist.

Zur Optimierung der Verschlußwirkung sieht eine bevorzugte Ausführungsform der Erfindung vor, daß der Deckel in der geschlossenen Stellung an der Wand des Behälters haftet. Dieses kann beispielsweise dadurch erreicht werden, daß auf einer der Einwurföffnung zugewandten Innenseite des Deckels und/oder auf der der Innenseite des Deckels gegenüberliegenden Außenfläche der Wand des Behälters ein Haftmaterial aufgebracht ist, zum Beispiel umlaufend oder nur punktweise.

Zur verbesserten Vermeidung einer Geruchsbelästigung sieht eine Ausgestaltung der Erfindung vor, daß an dem Deckel ein Geruchsmittel zum Erzeugen eines Frischegeruchs angeordnet ist. Bei dem Geruchsmittel kann es sich beispielsweise um ein Vlies handeln, das mit einem Geruchsdeo getränkt ist.

Der Hygienestandard kann weiter dadurch verbessert werden, daß die Einwurföffnung mit einer Abdeckung mit einem Einwurfschlitz bedeckt ist. Hierdurch kann die Einwurföffnung nicht nur blickschützend abgedeckt werden, auch das Entweichen von Gerüchen aus dem Aufnahmeraum wird mit Hilfe der Abdeckung unterbunden.

Vorteilhaft ist die Abdeckung mit Hilfe von Streifen aus einem nachgebenden Material gebildet, die an der Wand des Behälters befestigt sind und sich im Bereich des Einwutfschlitzes überlappen. Hierdurch ist auf einfache Weise eine Abdeckung gebildet, die einerseits blickschützend ausbildbar ist und andererseits einen bequemen Einwurf des verpackten und zu entsorgenden Hygieneartikels in den Aufnalumeraum gewährleistet. Eine Ausgestaltung der Erfindung kann vorsehen, daß die Streifen Foliestreifen sind, so daß die Streifen aus einem Hygienestandards genügenden Material sind.

Zum Aufstellen des Behälters mit dem Aufnahmeraum und dem Vorratsraum ist bei einer bevorzugten Ausführungsform vorgesehen, daß der Behälter lösbar in einer Umverpackung angeordnet ist. Die Umverpackung kann dazu genutzt werden, den Behälter stehend oder hängend an dem gewünschten Ort anzubringen, zum Beispiel in einer Toilette. Die Umverpackung kann beispielsweise aus Edelstahl oder Kunststoff sein und ein modisches Design aufweisen, um eine eher schlichte und kostengünstige Ausgestaltung des Behälters zu kaschieren. Darüber hinaus ist es mit Hilfe der Umverpackung möglich, das Aussehen der aufgestellten Vorrichtung an die Gestaltung des Aufstellraums anzupassen, zum Beispiel eine bestimmte farbliche Gestaltung von Wandfliesen.

Die Bereitstellung von frischen Hygieneartikeln wird bei einer bevorzugten Fortbildung der Erfindung dadurch erreicht, daß ein weiterer Vorratsraum für frische Hygieneartikel vorgesehen ist, welcher von dem Aufnahmeraum und dem Vorratsraum getrennt ist. Der weitere Vorratsraum ist zweckmäßig in einem von dem Behälter getrennten weiteren Behälter gebildet, welcher seinerseits lösbar in der Umverpackung angeordnet sein kann. Auf diese Weise ist es ermöglicht, den Behälter mit dem Aufnahmeraum und dem Vorratsraum auszutauschen und den weiteren Vorratsbehälter mit frischen Hygieneartikeln noch zu nutzen, wenn in dem weiteren Vorratsraum noch verbleibende frische Hygieneartikel vorhanden sind, der Aufnahmeraum aber bereits mit zu entsorgenden Hygieneartikeln vollständig gefüllt ist. Es kann alternativ vorgesehen sein, den weiteren Vorratsraum in dem Behälter zu bilden.

Eine bequeme Entnahme der frischen Hygieneartikel wird bei einer zweckmäßigen Fortbildung der Erfindung dadurch erreicht, daß in dem Vorratsraum ein zu einer Öffnung hin abfallender Boden gebildet ist. Hierdurch rutschen in dem Vorratsraum noch befindliche frische Hygieneartikel stets zur Öffnung hin und sind so für den Benutzer ohne weiteres durch die Öffnung greifbar.

Eine kostengünstige Herstellung und eine umweltschonende Entsorgung wird bei einer vorteilhaften Ausführungsform der Erfindung dadurch unterstützt, daß der Behälter und/oder der weitere Behälter aus einem Papp- oder einem Verbundmaterial sind. Hierbei kann zur Verbesserung der Hygienestandards vorgesehen sein, daß das Papp- oder das Verbundmaterial zumindest im Bereich von Innenwänden des Aufnahmeraums beschichtet ist, beispielsweise mit einer flüssigkeitsabweisenden Beschichtung.

Zweckmäßig sind in dem Vorratsraum mehrere Verpackungen gelagert, die einzeln nacheinander entnehmbar sind. Je nach Bedarf können so eine oder mehrere Verpackungen entnommen werden. Hierbei kann vorgesehen sein, daß benachbarte der mehreren Verpackungen lösbar miteinander verbunden sind, wie dieses beispielsweise für Plastiktüten auf einer Rolle oder im gefalteten Zustand bekannt ist. Eine weitere Verbesserung der hygienischen Bedingungen wird dadurch erreicht, daß die mehreren Verpackungen Kunststofftüten sind.

Die Einwurföffnung kann im Bereich einer oberen Deckfläche des Behälters oder auf einer Frontseite des Behälters angeordnet sein. Die Anordnung der Einwurföffnung im Bereich der oberen Deckfläche des Behälters hat den Vorteil, daß hierdurch die Handhabung erleichtert wird, nämlich der Einwurf des zu entsorgenden Hygieneartikels und gegebenenfalls das Öffnen und Schließen des Deckels für die Einwurföffnung. Bei der Anordnung der Einwurföffnung auf der Frontseite des Behälters besteht der Vorteil, daß bei dieser Ausführungsform die Einwurföffnung unterhalb der Entnahmeöffnung angeordnet werden kann.

Die Erfindung wird im folgenden anhand von Ausführungsbeispielen unter Bezugnahme auf eine Zeichnung näher erläutert. Hierbei zeigen:
- Fig. 1: einen Behälter und eine Umverpackung zur Aufnahme des Behälters;

- Fig. 2: den Behälter nach Fig. 1 nach dem Anordnen in der Umverpackung;
- Fig. 3: den Behälter nach Fig. 1 mit einem geöffneten Deckel sowie einen weiteren Behälter für frische Hygieneartikel;
- Fig. 4A: eine weitere Ausführungsform eines Behälters in einer Umverpackung;
- Fig. 4B: einer Teildarstellung des Behälters nach Fig. 4A;
- Fig. 5: eine andere Ausführungsform eines Behälters in einer Umvcrpackung;
- Fig. 6A: einen Behälter in einer Umverpackung mit einem Schubladen fach; und
- Fig. 6B: den Behälter nach Fig. 6B mit geöffnetem Schubladenfach.

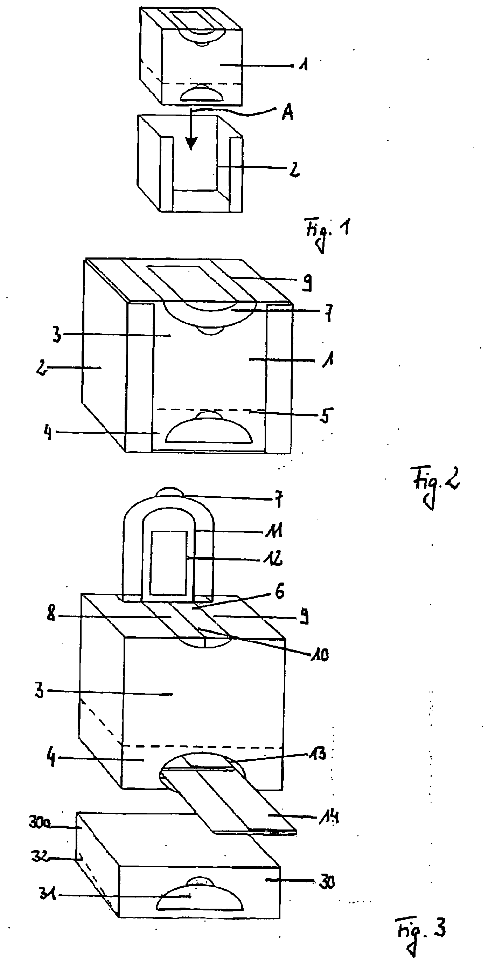

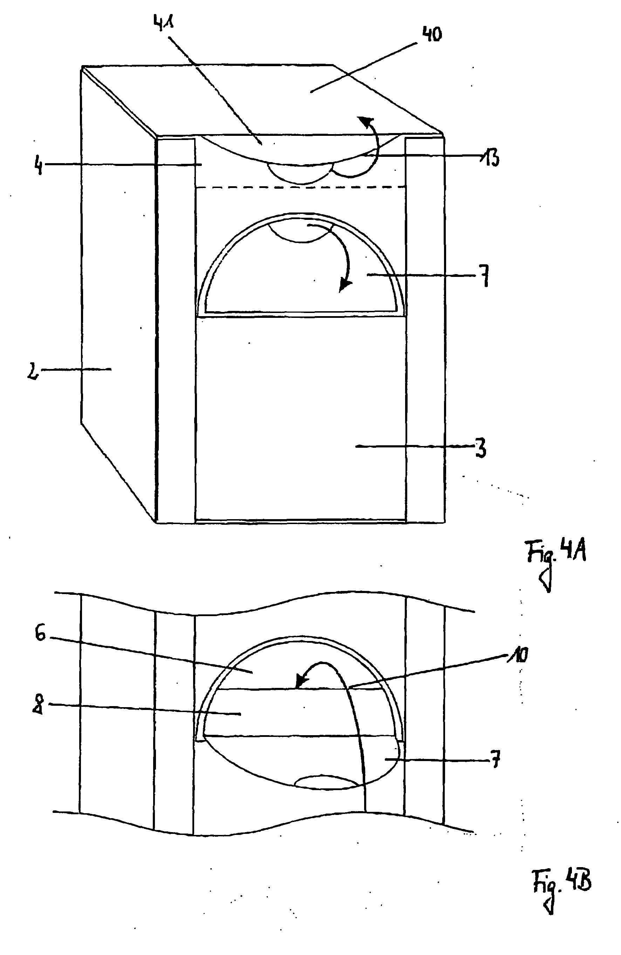

Fig. 1 zeigt eine perspektivische Darstellung eines Behälters 1 sowie einer Umverpackung 2 zum Aufnehmen des Behälters 1, was mit Hilfe eines Pfeils A schematisch dargestellt ist. Die Umverpackung 2 dient dazu, den Behälter 1 an dem gewünschten Ort, zum Beispiel in einem Bad oder einer Toilette, stehend oder an der Wand hängend zu lagern. Zu diesem Zweck kann die Umverpackung 2 zum Beispiel aus Edelstahl oder Kunststoff sein und mit einem modischen Design versehen werden.

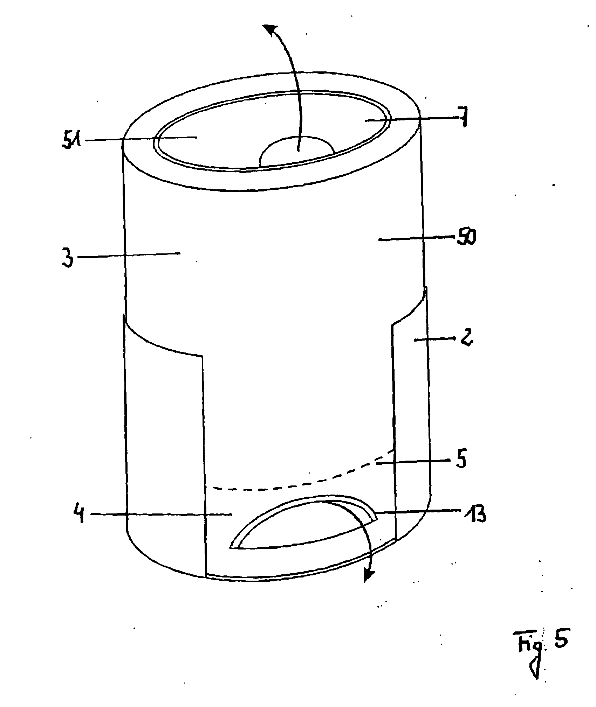

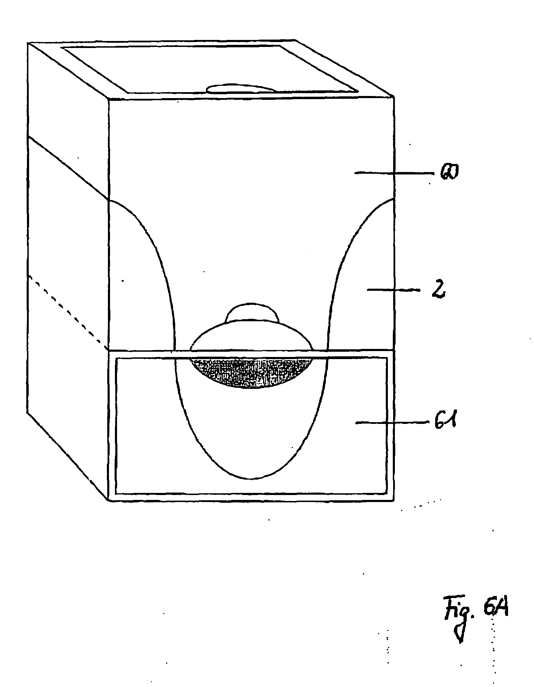

Fig. 2 zeigt eine perspektivische Darstellung des Behälters 1 nach Fig. 1 nach dem Anordnen in der Umverpackung 2. In dem Behälter 1 sind ein Aufnahmeraum 3 sowie ein Vorratsraum 4 gebildet, die mittels eines Zwischenbodens 5 voneinander getrennt sind. Der Aufnahmeraum 3 dient zum Aufnehmen gebrauchter Hygieneartikel, beispielsweise Damenbinden oder Windeln, die durch eine Einwurföffnung 6 (vgl. Fig. 3) in den Aufnahmeraum 3 eingeworfen werden. Die Einwurföffnung 6 ist bei der Darstellung nach Fig. 2 durch einen Deckel 7 abgedeckt.

Fig. 3 zeigt eine perspektivische Darstellung des Behälters 1 nach Fig. 1 mit geöffnetem Dekkel 7. Im Bereich der Einwurföffnung 6 sind Foliestreifen 8 auf einer Innenseite einer Wand 9 des Behälters 1 angeklebt. Die Foliestreifen 8 überlappen sich im Bereich eines Einwurfschlitzes 10, durch den die zu entsorgenden Hygieneartikel in den Aufnahmeraum 3 eingeworfen werden können.

Auf einer Unterseite 11 des Deckels 7 ist ein Flies 12 angeordnet, welches mit einem Geruchsdeo getränkt ist.

Unterhalb des Aufnahmeraums 3 ist der Vorratsraum 4 gebildet, welcher seinerseits von außen durch eine Entnahmeöffnung 13 zugänglich ist. Durch die Entnahmeöffnung 13 kann eine Verpackung 14 herausgezogen werden, die dazu dient, den zu entsorgenden Hygieneartikel vor dem Einwerfen in den Aufnahmeraum 3 aufzunehmen. Bei der Verpackung 14 handelt es sich beispielsweise um eine Kunststofftüte, die aus einem blickdichten Kunststoffmaterial gebildet sein kann. In dem Vorratsraum 4 sind mehrere Verpackungen der gleichen Art angeordnet, die bei Bedarf einzeln durch die Entnahmeöffnung 13 herausgenommen werden können. Die in dem Vorratsraum 4 gelagerten Verpackungen sind so miteinander verbunden, daß eine herausgenommene Verpackung von noch in dem Vorratsraum 4 verbleibenden Verpakkungen ohne weiteres getrennt werden kann, beispielsweise mit Hilfe punktförmiger Verbindingen zwischen Kanten von Plastiktüten.

In Fig. 3 ist darüber hinaus ein weiterer Behälter 30 dargestellt, welcher als Reservoir für frische Hygieneartikel einer weiteren Vorratsraum 30a aufweist. Durch eine Öffnung 31 kann ein frischer Hygieneartikel entnommen werden. Der weitere Behälter 30 weist einen zu der Öffnung 31 hin abfallenden Boden 32 auf, so daß frische Hygieneartikel bequem entnommen werden können.

Fig. 4 zeigt eine perspektivische Darstellung einer weiteren Ausführungsform eines Behälters 40 zum Aufnehmen eines zu entsorgenden Hygieneartikels. In Fig. 4 werden für gleiche Merkmale gleiche Bezugszeichen wie in den Fig. 1 bis 3 verwendet. Die Einwurföffnung 6 ist auf einer Frontseite des Behälters 40 und unterhalb der Entnahmeöffnung 13 für die Verpakkungen gebildet, die ihrerseits mit einem Deckel 41 verschließbar ist. Gemäß Fig. 4B, die einen Ausschnitt des Behälters 40 nach Fig. 4A zeigt, ist die Einwurföffnung 6 wiederum mit Foliestreifen 8 bedeckt.

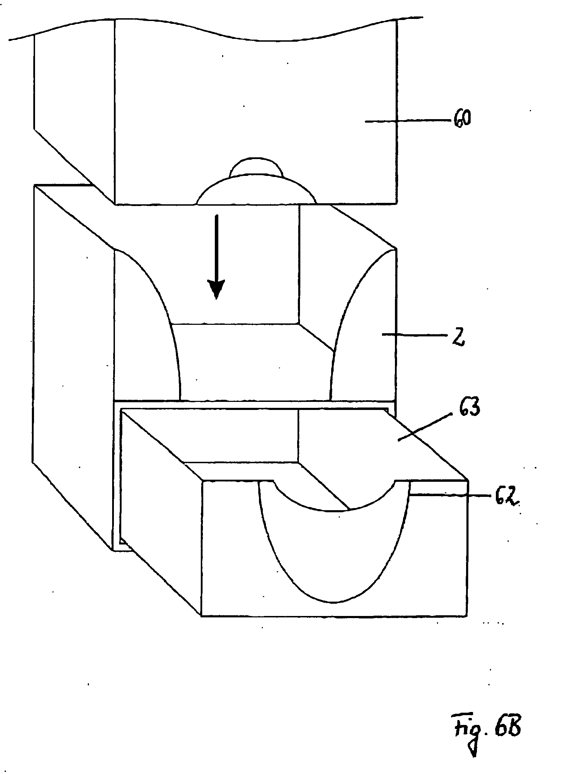

Fig. 5 zeigt eine andere Ausführungsform eines Behälters 50 zur Aufnahme eines zu entsorgenden Hygieneartikels. In Fig. 5 werden für gleiche Merkmale gleiche Bezugszeichen wie in den Fig. 1 bis 3 verwendet. Der Deckel 7 zum Abdecken der Einwurföffnung 6 ist bei der Ausführungsform nach Fig. 5 im Bereich einer oberen Deckfläche 51 des Behälters 50 angeordnet. Der Vorratsraum 4 ist unterhalb des Aubahmeraums 3 angeordnet.

Die Fig. 6A und 6B zeigen eine Ausführungsform eines Behälters 60 zur Aufnahme eines zu entsorgendenen Hygieneartikels. In den Fig. 6A und 6B werden für gleiche Merkmale gleiche Bezugszeichen wie in den Fig. 1 bis 3 verwendet. Der Behälter 60 ist in der Umverpackung 2 angeordnet, die einerseits zur Aufnahme des Behälters 60 dient und andererseits zum Befestigen des Behälters 60 an der Wand genutzt werden kann, weshalb die Umverpackung 2 auch als Halterung bezeichnet werden kann. Zu diesem Zweck ist die Umverpackung 2 vorzugsweise aus Edelstahl oder Kunststoff mit Befestigungsmöglichkeiten an der Rückwand oder im Boden, beispielsweise in Form von Durchbrüchen, in die Schrauben eingeführt werden können, zum Beispiel zur Befestigung der Umverpackung 2 mittels Dübel-Schraubenverbindungen. Unterhalb des Behälters 60 ist in der Umverpackung/der Halterung 2 ein Schubfach 61 gebildet, welches zur Aufnahme neuer Tampons nutzbar ist. Eine Vorderwand 62 einer Schublade 63 kann, aus einem transparenten Material sein, um eine Kontrolle der Füllmenge in der Schublade 61 zu ermöglichen.

Wie sich aus Fig. 6B ergibt, ist der Behälter 60 lösbar in der Umverpackung 2 angeordnet.

## Patentansprüche

1. Vorrichtung zum Aufnehmen eines zu entsorgenden Hygieneartikels, insbesondere Damen-Tampon, mit einem Behälter (1; 40; 50; 60) aus einem recyclebaren Material, in dem ein Aufnahmeraum (3) für den Hygieneartikel gebildet ist, wobei in einer Wand (9) des Behälters (1; 40; 50; 60) eine verschließbare Einwurföffnung (6), durch die der Hygieneartikel in den Aufnahmeraum (3) eingeworfen werden kann, gebildet ist, **dadurch gekennzeichnet, daß** ein von dem Aufnahmeraum (3) getrennter Vorratsraum (4) mit Verpackungen (14) zur Aufname des Hygieneartikels für eine Entsorgung und eine Entnahmeöffnung (13) gebildet sind, durch die die Verpackungen (14) entnehmbar sind.

2. Vorrichtung nach Anspruch 1, **gekennzeichnet durch** einen Deckel (7), der zum Verschließen und zum Freigeben der Einwurföffnung (6) zwischen einer geschlossenen und einer geöffneten Stellung schwenkbar ist.

3. Vorrichtung nach Anspruch 2, **dadurch gekennzeichnet, daß** der Deckel (7) in der geschlossenen Stellung an der Wand (9) des Behälters (1; 40; 50; 60) haftet.

4. Vorrichtung nach Anspruch 1 oder 2, **dadurch gekennzeichnet, daß** an dem Deckel (7) ein Geruchsmittel zum Erzeugen eines Frischegeruchs angeordnet ist.

5. Vorrichtung nach einem der vorangehenden Ansprüche, **dadurch gekennzeichnet, daß** die Einwurföffnung (6) mit einer Abdeckung (8) mit einem Einwurfschlitz (10) bedeckt ist.

6. Vorrichtung nach Anspruch 5, **dadurch gekennzeichnet, daß** die Abdeckung (8) mit Hilfe von Streifen aus einem nachgebenden Material gebildet ist, die an der Wand (9) des Behälters (1; 40; 50; 60) befestigt sind und sich im Bereich des Einwurfschlitzes (10) überlappen.

7. Vorrichtung nach Anspruch 6, **dadurch gekennzeichnet, daß** die Streifen Foliestreifen sind.

8. Vorrichtung nach einem der vorangehenden Ansprüche, **dadurch gekennzeichnet, daß** der Behälter (1; 40; 50; 60) lösbar in einer Umverpackung (2) angeordnet ist.

9. Vorrichtung nach einem der vorangehenden Ansprüche, **gekennzeichnet durch** einen weiteren Vorratsraum (30a; 61) für frische Hygieneartikel, welcher von dem Aufnahmeraum (3) und dem Vorratsraum (4) getrennt ist.

10. Vorrichtung nach Anspruch 9, **dadurch gekennzeichnet, daß** in dem weiteren Vorratsraum (30a) ein zu einer Öffnung (31) hin abfallender Boden (32) gebildet ist.

11. Vorrichtung nach Anspruch 9 oder 10, **dadurch gekennzeichnet, daß** der weitere Vorratsraum (30a) in einem von dem Behälter (1; 40; 50; 60) getrennten weiteren Behälter (30) gebildet ist, welcher lösbar in der Umverpackung (2) angeordnet ist.

12. Vorrichtung nach Anspruch 11, **dadurch gekennzeichnet, daß** der weitere Behälter (61) als Schublade (63) gebildet ist.

13. Vorrichtung nach einem der vorangehenden Ansprüche, **dadurch gekennzeichnet, daß** der Behälter (1; 40; 50; 60) und/oder der weitere Behälter (30) aus einem Papp- oder einem Verbundmaterial sind.

14. Vorrichtung nach Anspruch 13, **dadurch gekennzeichnet, daß** das Papp- oder das Verbundmaterial zumindest im Bereich von Innenwänden des Aufnahmeraums (3) beschichtet ist.

15. Vorrichtung nach einem der vorangehenden Ansprüche, **dadurch gekennzeichnet, daß** in dem Vorratsraum (4) mehrere Verpackungen gelagert sind, die einzeln nacheinander entnehmbar sind.

16. Vorrichtung nach Anspruch 15, **dadurch gekennzeichnet, daß** benachbarte der mehreren Verpackungen lösbar miteinander verbunden sind.

17. Vorrichtung nach Anspruch 15 oder 16, **dadurch gekennzeichnet, daß** die mehreren Verpackungen Kunststofftüten sind.

18. Vorrichtung nach einem der vorangehenden Ansprüche, **dadurch gekennzeichnet, daß** die Einwurföffnung (6) im Bereich einer oberen Deckfläche (51) des Behälters (50) angeordnet ist.

19. Vorrichtung nach einem der Ansprüche 1 bis 17, **dadurch gekennzeichnet, daß** die Einwurföffnung (6) auf einer Frontseite des Behälters (1; 40) angeordnet ist.

## Claims

1. A device for receiving an article of personal hygiene to be disposed of, in particular a sanitary tampon, comprising a receptacle (1; 40; 50; 60) made of recycling material and formed with a hold space (3) for the article and a storage space (4) with packings (14) which is separate from the hold space (3) and takes up the article for disposal, a wall (9) of the receptacle (1; 40; 50; 60) being formed with a dump opening (6) which is adapted to be closed and through which the article can be thrown into the hold space (3), **characterized by** a storage space (4) with packings (14) which is separate from the hold space (3) and takes up the article for disposal, and a withdrawal opening (13) through which the packings (14) can be taken out.

2. The device as claimed in claim 1, **characterized by** a lid (7) adapted to be swung between a closed and an open positions to block and unblock the dump opening (6), respectively.

3. The device as claimed in claim 2, **characterized in that** the lid (7), in closed position, adheres to the wall (9) of the receptacle (1; 40; 50; 60).

4. The device as claimed in claim 1 or 2, **characterized in that** a fragrant substance is disposed on the lid (7) to produce a smell of freshness.

5. The device as claimed in any one of the preceding claims, **characterized in that** the dump opening (6) is covered by a cover (8) which is formed with a slit (10).

6. The device as claimed in claim 5, **characterized in that** the cover (8) is defined by strips of resilient material which are fastened to the wall (9) of the receptacle (1; 40; 50; 60) and overlap in the area of the slit (10).

7. The device as claimed in claim 6, **characterized in that** the strips are strips of sheet material.

8. The device as claimed in any one of the preceding claims, **characterized in that** the receptacle (1; 40; 50; 60) is arranged releasably in an outer packing (2).

9. The device as claimed in any one of the preceding claims, **characterized by** another storage space (30a, 61) for fresh articles of personal hygiene, said storage space being separate from the hold space (3) and the storage space (4).

10. The device as claimed in claim 9, **characterized in that** the other storage space (30a) is formed with a bottom (32) which is inclined towards an aperture (31).

11. The device as claimed in claim 9 or 10, **characterized in that** the other storage space (30a) is formed in another receptacle (30) which is separate from the receptacle (1; 40; 50; 60) and arranged releasably in the outer packing (2).

12. The device as claimed in claim 11, **characterized in that** the other receptacle (61) is embodied by a drawer (63).

13. The device as claimed in any one of the preceding claims, **characterized in that** the receptacle (1; 40; 50; 60) and/or the other receptacle (30) are made of cardboard or composite material.

14. The device as claimed in claim 13, **characterized in that** the cardboard or composite material is coated at least in the area of interior walls of the hold space (3).

15. The device as claimed in any one of the preceding claims, **characterized in that** a plurality of packings are stored in the storage space (4) and to be withdrawn individually one after the other.

16. The device as claimed in claim 15, **characterized in that** adjacent ones of the plurality of packings are separably linked.

17. The device as claimed in claim 15 or 16, **characterized in that** the plurality of packings are plastic bags.

18. The device as claimed in any one of the preceding claims, **characterized in that** the dump opening (6) is provided in the area of a top covering surface (51) of the receptacle (50).

19. The device as claimed in any of claims 1 to 17, **characterized in that** the dump opening (6) is provided at a front side of the receptacle (1; 40).

## Revendications

1. Dispositif servant à recevoir un article hygiénique à jeter, en particulier un tampon périodique, ledit dispositif comprenant un récipient (1 ; 40 ; 50 ; 60) réalisé dans un matériau recyclable, récipient dans lequel est formé un espace de réception (3) pour l'article hygiénique où, dans une paroi (9) du récipient (1 ; 40 ; 50 ; 60), est formée une ouverture d'introduction (6) pouvant être fermée, à travers laquelle l'article hygiénique peut être jeté dans l'espace de réception (3),
**caractérisé en ce que** sont formés, un espace de stockage (4) séparé de l'espace de réception (3), ledit espace de stockage comprenant des emballages (14) et servant à recevoir l'article hygiénique prévu pour être jeté, et une ouverture de distribution (13) à travers laquelle les emballages (14) peuvent être retirés.

2. Dispositif selon la revendication 1, **caractérisé par** un couvercle (7) qui, pour fermer et pour dégager l'ouverture d'introduction (6), peut pivoter entre une position fermée et une position ouverte.

3. Procédé selon la revendication 2, **caractérisé en ce que** le couvercle (7), en position fermée, s'applique sur la paroi (9) du récipient (1 ; 40 ; 50 ; 60).

4. Dispositif selon la revendication 1 ou 2, **caractérisé en ce qu'**un produit odorisant servant à donner un parfum de fraîcheur est disposé sur le couvercle (7).

5. Dispositif selon l'une quelconque des revendications précédentes, **caractérisé en ce que** l'ouverture d'introduction (6) est recouverte par un élément de recouvrement (8) comprenant une fente d'introduction (10).

6. Dispositif selon la revendication 5, **caractérisé en ce que** l'élément de recouvrement (8) est formé à l'aide de bandes réalisées dans un matériau élastique, bandes qui sont fixées sur la paroi (9) du récipient (1 ; 40 ; 50 ; 60) et se chevauchent dans la zone de la fente d'introduction (10).

7. Dispositif selon la revendication 6, **caractérisé en ce que** les bandes sont des bandes de feuilles.

8. Dispositif selon l'une quelconque des revendications précédentes, **caractérisé en ce que** le récipient (1 ; 40 ; 50 ; 60) est disposé de manière amovible dans un élément enveloppant (2).

9. Dispositif selon l'une quelconque des revendications précédentes, **caractérisé par** un autre espace de stockage (30a ; 61) pour des articles hygiéniques frais, autre espace de stockage qui est séparé de l'espace de réception (3) et de l'espace de stockage (4).

10. Dispositif selon la revendication 9, **caractérisé en ce que**, dans l'autre espace de stockage (30a), est formé un fond (32) incliné vers une ouverture (31).

11. Dispositif selon la revendication 9 ou 10, **caractérisé en ce que** l'autre espace de stockage (30a) est formé dans un autre récipient (30) séparé du récipient (1 ; 40 ; 50 ; 60), autre récipient qui est disposé en étant amovible dans l'élément enveloppant (2).

12. Dispositif selon la revendication 11, **caractérisé en ce que** l'autre récipient (61) est formé comme un tiroir (63).

13. Dispositif selon l'une quelconque des revendications précédentes, **caractérisé en ce que** le récipient (1 ; 40 ; 50 ; 60) et/ou l'autre récipient (30) sont réalisés dans un matériau en carton ou un matériau composite.

14. Dispositif selon la revendication 13, **caractérisé en ce que** le matériau en carton ou le matériau composite est recouvert au moins dans la zone de parois intérieures de l'espace de réception (3).

15. Dispositif selon l'une quelconque des revendications précédentes, **caractérisé en ce que** plusieurs emballages sont placés dans l'espace de stockage (4), emballages qui peuvent être retirés individuellement l'un après l'autre.

16. Dispositif selon la revendication 15, **caractérisé en ce que** plusieurs emballages voisins sont assemblés les uns avec les autres, de manière détachable.

17. Dispositif selon la revendication 15 ou 16, **caractérisé en ce que** les emballages multiples sont des sacs en matière plastique.

18. Dispositif selon l'une quelconque des revendications précédentes, **caractérisé en ce que** l'ouverture d'introduction (6) est disposée dans la zone d'une surface de couverture supérieure (51) du récipient (50).

19. Dispositif selon l'une quelconque des revendications 1 à 17, **caractérisé en ce que** l'ouverture d'introduction (6) est disposée sur une face frontale du récipient (1 ; 40).
